# EUROPEAN PATENT APPLICATION

(11) **EP 4 000 687 A1**
(43) Date of publication of application: **25.05.2022**
(21) Application number: 21202800.5
(22) Date of filing: 15.10.2021
(51) Int. Cl.: A61N 7/00, A61B 90/50

(54) **LOW-INTENSITY FOCUSED ULTRASOUND TREATMENT APPARATUS**

(30) Priority: 18.11.2020 KR 20200154994
(71) Applicant: Neurosona Co., Ltd., Songpa-gu Seoul 05836 (KR)
(72) Inventor: Cho, Nam Kuy, 16944 Gyeonggi-do (KR); Kim, Jin Su, 16884 Gyeonggi-do, (KR); Yoo, Min Su, 05310 Seoul (KR); Yang, Yun Seob, 14923 Gyeonggi-do (KR)
(74) Representative: Grünecker Patent- und Rechtsanwälte PartG mbB

(57) **Abstract**

A technology related to a low intensity focused ultrasonic treatment device is disclosed. According to an aspect of the proposed invention, a single-element ultrasound transducer having a focused region Focal length and output strength characteristics that can safely treat a patient's deep brain is applied to a low intensity focused ultrasonic treatment device.

## Description

### [Technical field]

A technology related to a low-intensity focused ultrasound treatment device is disclosed.

### [Technology behind the invention]

Ultrasound stimulation therapy that can stimulate brain lesions without a physical invasive process is known. As is well known, among the cranial nerve stimulation techniques, the transcranial direct current stimulation technique or the transcranial magnetic stimulation technique has a problem that it is difficult to treat deep brain and cannot treat a precise area.

Low intensity focused ultrasound technology can safely activate or inhibit neurons' activity in specific areas of the deep brain without side effects. Through such cranial nerve control, low-intensity focused ultrasound technology can treat mental disorders such as depressive disorder and schizophrenia and chronic nervous system diseases such as Alzheimer's and epilepsy. The low-intensity focused ultrasound technology induces stable cavitation for microbubbles, thereby temporarily opening the cerebrovascular barrier. Accordingly, various therapeutic agents for brain diseases can be delivered to the brain.

However, in implementing the low-intensity focused ultrasound treatment apparatus, conventionally, a so-called multi-array transducer in which multiple small transducers is arranged has been used. To control the positions of multiple ultrasonic focusing regions, the beamforming control algorithm applies voltages having different amplitudes or phases to each of the transducer elements forming the array.

However, according to this multi-array transducer technology, more than 1000 or more than 500 small transducers are required, and ultrasonic application equipment (e.g., ultrasonic generator, linear amplifier, electric signal generator, waveform modulator, wiring unit, etc.) becomes relatively large. Also, wiring according to drive signals (e.g., amplitude, phase control) becomes complicated for each transducer. Further, the circuit for driving becomes complicated, and the driving control algorithm and the error control algorithm are also complicated. Accordingly, there is a problem that the cost of manufacturing the low-intensity focused ultrasound treatment apparatus increases.

According to the multi-array transducer technology, it is possible to focus on multiple regions using a single multi-array transducer by using phase-modulated beam forming. There is a problem that the location is changed. That is, a multi-array transducer places many small transducers in a semicircular structure larger than the brain. When targeting the brain's cortical part, for example, the upper part of the brain, the angle for ultrasonic incidence is not made to the target for the transducer located in a specific part of the multi-array. Accordingly, the number of multi-array transducers that transmit effective ultrasonic energy is reduced, resulting in a difficult problem in that effective ultrasonic application.

Meanwhile, for the low-intensity focused ultrasound beam to pass through the patient's skull and be delivered to the set focus area, the ultrasonic transducer and the patient's skull must be in close contact with each other and fixed. Conventionally, to closely contact and fix the ultrasound transducer and the patient's skull, a skull positioning device has substantially fixed the patient's skull in a single position. Accordingly, the patient cannot move his/her head at all by the skull position fixing device during the ultrasound treatment . Therefore, the patient may feel uncomfortable.

### [Contents of the invention]

### [Problem to be solved]

The proposed invention aims to provide a low-intensity focused ultrasound treatment apparatus capable of reducing manufacturing and maintenance costs.

Another purpose of the proposed invention is to provide a low-intensity focused ultrasound treatment apparatus. An effective ultrasound focusing region does not differ depending on the treatment site. Furthermore, another purpose of the proposed invention is to provide a low-intensity focused ultrasound treatment apparatus in which an ultrasonic application device can be simplified.

Another purpose of the proposed invention is to provide a low-intensity focused ultrasound treatment apparatus. A single-element ultrasound transducer capable of precisely positioning various treatment areas of a deep brain region is applied.

Another purpose of the proposed invention is to provide a low-intensity focused ultrasound treatment apparatus capable of adjusting the position and various postures of a single-element ultrasound transducer concerning a patient's head.

Another purpose of the proposed invention is to provide a low-intensity focused ultrasound treatment apparatus comprising an ultrasonic transducer capable of slightly moving a patient's head during ultrasound treatment and a contact and fixation configuration between the patient's skull.

### [Means to solve the problem]

According to an aspect of the proposed invention, a single-element ultrasound transducer with a focus area, a focus distance, and an output intensity characteristic capable of safe treatment for a patient's deep brain are applied to low-intensity focused ultrasound treatment apparatus.

Further, according to a further aspect of the proposed invention, a single-element ultrasonic transducer applied to a low-intensity focused ultrasound treatment device is plural for each focusing distance. One of multiple single-element ultrasonic transducers having a corresponding focusing distance can be replaced. The device ultrasonic transducer has a detachable structure.

Further, according to the proposed invention's further aspect, an ultrasonic gel layer for adjusting the focusing distance is placed on the bottom of the single element ultrasonic transducer.

Further, according to an additional aspect of the proposed invention, multiple band hook portions are formed on a single element ultrasonic transducer, and multiple fixed band portions fixed to each of multiple band hook portions are formed in the headband portion mounted on the patient's skull. Further, according to a further aspect of the proposed invention, the articulated manipulation unit is detachably coupled to one of multiple single-element transducers. The articulated manipulation unit precisely adjusts the position and posture of the single element ultrasonic transducer.

Further, according to an additional aspect of the proposed invention, the free articulating Arm included in the multi-joint manipulation unit adjusts the height and front-rear position of the single element ultrasonic transducer, and the manipulation Arm is included in the multi-joint manipulation unit is used in the single element ultrasonic transducer that is detachable and rotatable in all directions.

### [Effects of the Invention]

According to the proposed invention, it is possible to provide a low-intensity focused ultrasound treatment apparatus that reduces manufacturing and maintenance costs.

Further, according to the proposed invention, it is possible to provide a low-intensity focused ultrasound treatment apparatus in which a difference in effective ultrasound focus does not occur.

Further, according to the proposed invention, it is possible to provide a low-intensity focused ultrasound treatment apparatus in which the ultrasonic application device can be simplified.

Further, according to the proposed invention, it is possible to provide a low-intensity focused ultrasound treatment apparatus. A single element ultrasound transducer capable of precisely positioning various treatment areas of a deep brain region is applied.

Further, according to the proposed invention, it is possible to provide a low-intensity focused ultrasound treatment apparatus capable of adjusting the position and various postures of a single-element ultrasound transducer concerning a patient's head.

Further, according to the proposed invention, it is possible to provide a low-intensity focused ultrasound treatment apparatus including an ultrasonic transducer capable of slightly moving a patient's head during ultrasound treatment and a configuration of close contact fixation between the patient's head.

### [Simple explanation of drawings]

Drawing 1 shows the focused ultrasound beam output characteristics of a single-element ultrasound transducer according to an embodiment.
Drawing 2 is a perspective view showing a structure in which an articulated operator and a single-element ultrasound transducer are combined according to an embodiment.
Drawing 3 is a cross-sectional view showing a single-element ultrasound transducer according to an embodiment.
Drawing 4 is a perspective view showing a low intensity focused ultrasonic treatment device according to an embodiment.
Drawing 5a and Drawing 5b show a single-element ultrasound transducer and a headband mounting structure for closely contacting and fixing a patient's head according to an embodiment.
Drawing 6 shows the use of a single-element ultrasound transducer according to a change in the appearance of a semi-solid ultrasound gel layer according to an embodiment.

### [Specific contents for implementing the invention]

The above-described and additional aspects are embodied through embodiments described concerning the accompanying drawings. It is understood that the constituent elements of the respective embodiments may be variously combined within the embodiments unless otherwise stated or contradictory to each other.

### [Description of Claim 1 and Claim 2]

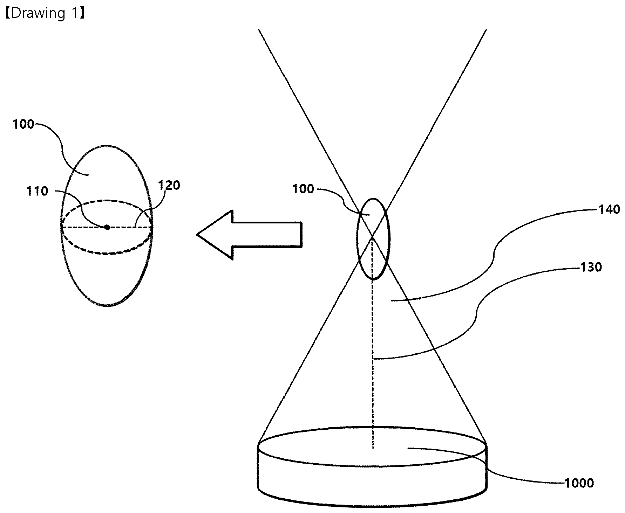

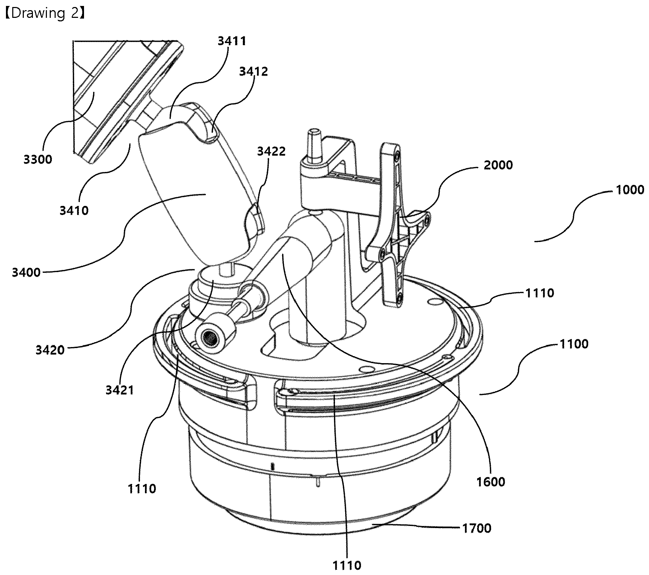

Drawing 1 shows the focused ultrasound beam output characteristics of a single-element ultrasound transducer according to an embodiment. Drawing 2 shows a structure in which an articulated operator and a single-element ultrasound transducer are combined according to an embodiment. It is a perspective view and will be described below concerning the above drawings.

As shown in Drawing 1, in the low intensity focused ultrasonic treatment device according to an aspect of the present invention, the focusing region diameter (120) at the maximum acoustic intensity point (110) in the focal region (100) is 6mm to 9mm; an ultrasound transducer (1000) outputting a low intensity focused ultrasound beam (140) having a focal length (130) of 30 mm to 80 mm from the surface (1000) to the maximum acoustic intensity point (110), and a single-element ultrasound transducer (1000), and includes a transducer marker (2000)that provides position and posture information of the single-element ultrasound transducer (1000).

According to an aspect of the present invention, a single-element ultrasound transducer (1000) is applied to a low intensity focused ultrasonic treatment device. The single-element ultrasound transducer (1000) has a focused region (100), a focused region diameter (120), a Focal length (130), and low-intensity output strength characteristics that enable safe treatment of a patient's deep brain.

According to an aspect of the present invention, the focused region diameter (120) at the maximum acoustic intensity point (110) in the focused region (100) where the ultrasound beam output from the single-element ultrasound transducer (1000) is focused, and this is 6mm to 9mm. Also, the Focal length (130) from the surface of the single-element ultrasound transducer (1000) to the maximum acoustic intensity point (110) is 30mm to 80mm. The acoustic strength of the ultrasound beam output by the single-element ultrasound transducer (1000) is low intensity, so that safe treatment for the patient's deep brain is possible. In an embodiment, the acoustic strength (acoustic intensity) in the focused region (100) of the ultrasound beam output from the single-element ultrasound transducer (1000) maybe 50 watts per square centimeter (50 W/cm²) or less.

As shown in Drawing 2, according to an aspect of the present invention, the low intensity focused ultrasonic treatment device is fixed to the single-element ultrasound transducer (1000) to provide position and posture information of the single-element ultrasound transducer (1000). The transducer marker (2000) provides position and posture information of the single-element ultrasound transducer (1000). The location tracking device (not shown) included in the low intensity focused ultrasonic treatment device recognizes the spatial position and posture of the single-element ultrasound transducer (1000) provided by the transducer marker (2000) provides low intensity focused ultrasonic treatment. Position and posture may be displayed on a monitor (not shown) included in the device.

Accordingly, for example, the user may move the single-element ultrasound transducer (1000) to match the transducer focused region (100) to the treatment area inside the patient's brain.

Using transducer markers (reflector, coil, etc.), headgear/band markers (reflector, coil, etc.), passive tool/pinpoints, and position trackers (optical trackers, IR cameras, electro-magnetic trackers, etc.), a registration step to match the patient's physical space with a space-based on a three-dimensional image acquired from CT/MRI may be performed. After that, the single-element ultrasound transducer (1000) can be moved to match the transducer focused region (100) to the treatment area inside the patient's brain. The matching step of the image space and the actual space corresponds to a technique known in the art, and thus a more detailed description thereof will be omitted.

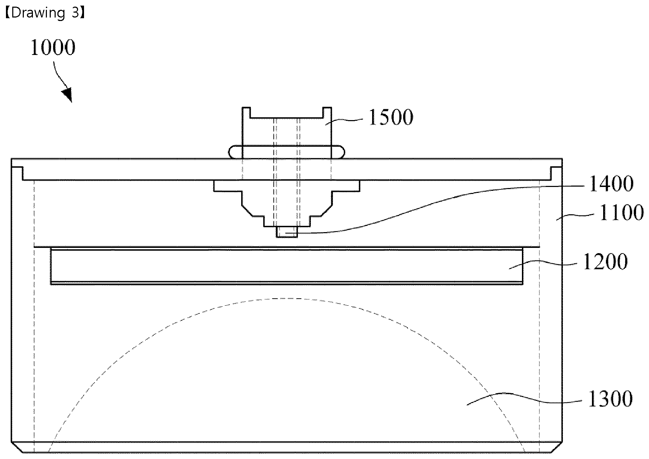

Drawing 3 is a cross-sectional view showing a single-element ultrasound transducer according to an embodiment and will be described concerning Drawing 3 below.

### [Description of Claim 3]

As shown in Drawing 3, according to an additional aspect, a single-element ultrasound transducer (1000) includes an electrode (1400) for applying a voltage, a single piezoelectric element (1200) for outputting a low-intensity ultrasound beam, spherical lens (1300) for focusing a low-intensity ultrasound beam, a connector (1500) for electrically connecting an external control cable (not shown) and an electrode (1400), a single piezoelectric element (1200), a spherical lens (1300), and a transducer housing (1100) in which the connector (1500) is placed.

In one embodiment, a single piezoelectric element (1200) outputting a low-intensity ultrasound beam, and a spherical lens (1300) focusing a low-intensity ultrasound beam below the output surface of the ultrasound beam of the single piezoelectric element (1200) are transducers. It is placed within the housing (1100). The ultrasound beam output's output characteristics by the single piezoelectric element (1200) and the spherical lens ((130)0) are as described in Drawing 1.

In an additional embodiment, the single-element ultrasound transducer (1000) may further include a connector cover (1600) for protecting the connector (1500) (see Drawing 2). The connector cover (1600) protects the portion of the connector (1500) exposed to the outside of the transducer housing (1100). Drawing 2 shows that the connector cover (1600) has an approximately "¬" shape and may have a through-hole therein. An external control cable (not shown) may be electrically connected to the connector (1500) through a through-hole

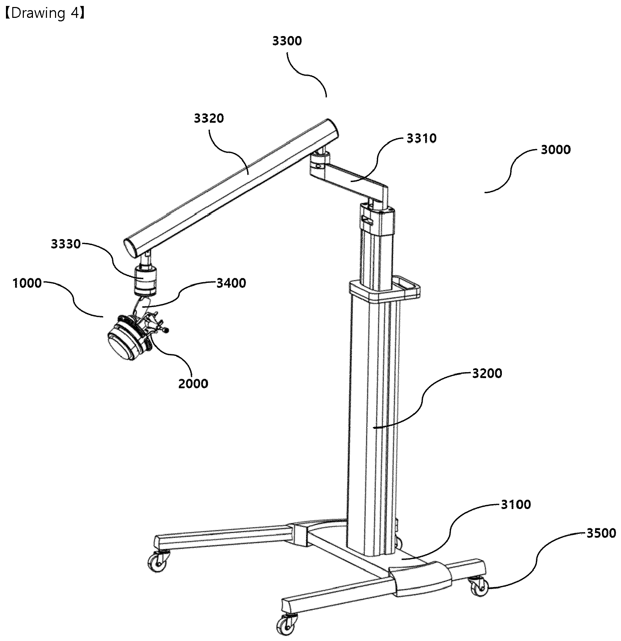

Drawing 4 is a perspective view showing a low intensity focused ultrasonic treatment device according to an embodiment. It will be described below in Drawing 4.

### [Description of Claim 4]

As shown in Drawing 4, according to an additional aspect, the low intensity focused ultrasonic treatment device is an articulated operator coupled to the single-element ultrasound transducer (1000) so that the position and posture of the single-element ultrasound transducer can be adjusted (3000) may be further included.

In one embodiment, the single-element ultrasound transducer (1000) may be coupled to an articulated operator (3000). The articulated operator (3000) includes multiple joints, and a medical practitioner can control multiple joints coupled to the single-element transducer (1000). Accordingly, the single-element ultrasound transducer position and posture (1000) concerning the patient's head can be variously adjusted.

In an embodiment, the articulated operator (3000) and the single-element transducer (1000) included in the low intensity focused ultrasonic treatment device may be fixedly coupled. Alternatively, the single-element transducer (1000) may be rotatably coupled to the articulated operator (3000). When rotatably coupled, the medical practitioner can further adjust the position and posture of the single-element transducer (1000) relative to the patient's head.

### [Description of Claim 5]

According to an additional aspect, the low-intensity ultrasound treatment device includes multiple single-element ultrasound transducers (1000) having different Focal lengths, and one of multiple single-element ultrasound transducers (1000) is an articulated operator (3000) may further include a detaching area (3400) (see Drawing 2).

As is well known, the single-element ultrasound transducer (1000) is limited in varying the Focal length (130), unlike the multi-array ultrasound transducer. In an embodiment, to correspond to multiple Focal lengths (130), multiple single-element ultrasound transducers (1000) may be required for each Focal length. In an embodiment, three single-element ultrasound transducers (1000) with a Focal length of 37mm, 70mm, and 80mm may be used in a low intensity focused ultrasonic treatment device.

In one embodiment, the detaching area (3400) may detachably couple one of multiple single-element ultrasound transducers (1000) to the articulated operator (3000). In an embodiment, the detaching area 340 is placed on the upper surface of the housing (1100) of the single-element ultrasound transducer (1000). It has a cylindrical part (not shown) with a spiral groove formed on the outer circumferential surface. An articulated operator (3000) is placed on the coupling surface of the helical protrusion is formed on the inner surface thereof; the helical protrusion may include a lid portion (not shown) helically coupled to the helical groove formed on the outer peripheral surface of the cylindrical part. Accordingly, for example, a single-element ultrasound transducer (1000) for a 37mm Focal length or a single-element ultrasound transducer (1000) for a 70mm Focal length may be selectively coupled to the articulated operator (3000).

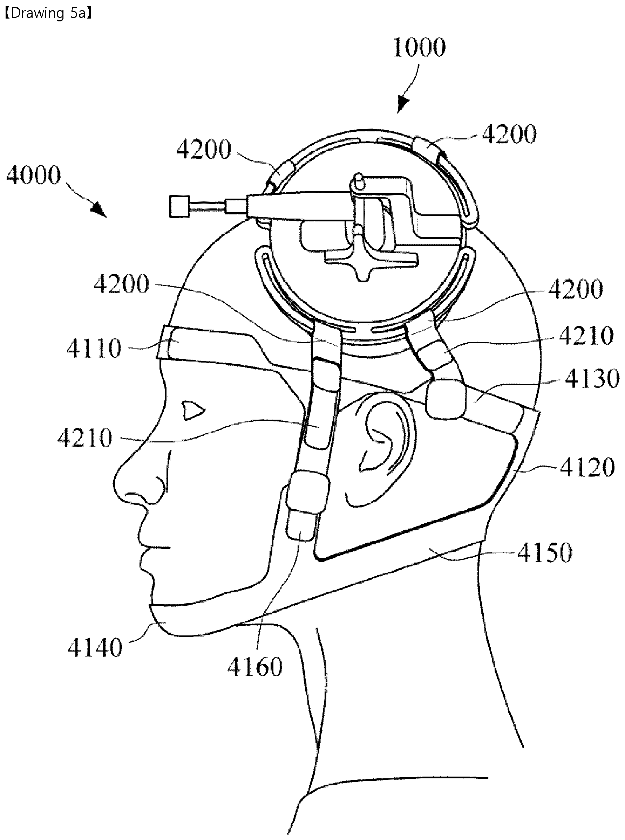

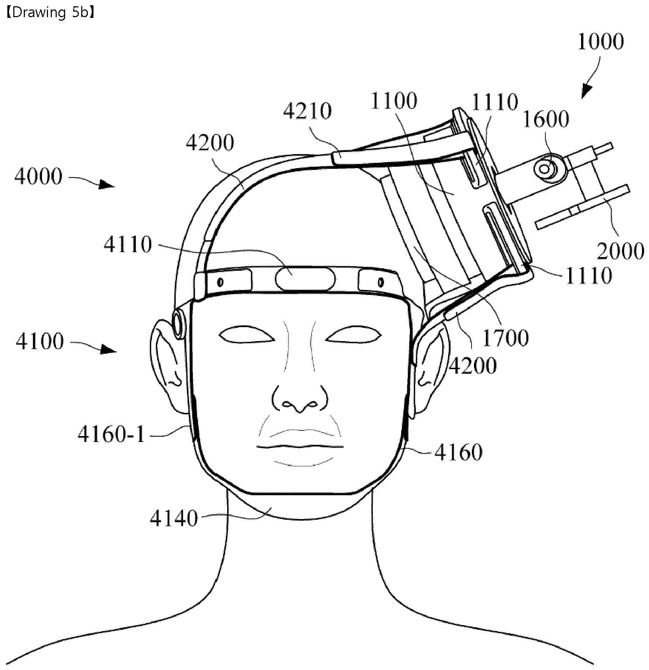

Drawing 5a and Drawing 5b show a headband mounting structure for intimately adhering and fixing a single-element ultrasound transducer and a patient's head according to an embodiment which will be described below in Drawing 5.

### [Description of Claim 6]

As shown in Drawing 5a and Drawing 5b, according to an additional aspect, the low intensity focused ultrasonic treatment device includes multiple band hangers (1110) formed along the edge of the single-element ultrasound transducer (1000). Multiple A headband (4000) including multiple bands fixed while surrounding the patient's head between the band hangers (1110) may be further included.

According to an additional aspect, multiple band hangers (1110) may be formed along the edge of the upper surface of the transducer housing (1100). In an embodiment, four grooves (1110) through which a band can be hooked may be formed on an upper surface of the transducer housing (1100). In an embodiment, the headband (4000) may include a headband body portion (4100) mounted on a patient's head and multiple fixed band portions (4200) fixed to multiple band hangers (1110). In an embodiment, when the patient wears the headband (4000) on his or her head, the headband body portion (4100) may closely support multiple portions of the patient's head. Multiple band portions supporting multiple head portions may be formed integrally in an embodiment.

In the embodiment, the headband body portion (4100) supports the forehead band portion (4110) supporting the head of the head, the band of lower jawbone (4140) supporting the jawbone lower jaw, the occipital region of the head. The occipital region band portion (4120), the upper left connecting band portion (4130) connecting the forehead band portion (4110)and the occipital region band portion (4120), the forehead band portion (4110)and the occipital region band portion (4120) ) To the upper right connection band portion (4130-1), the lower left connection band portion (4150) connecting the band of lower jawbone (4140) and the occipital region band portion (4120), and the band of lower jawbone of the left side of the jawbone vertically connecting the lower right connecting band portion (4150-1) connecting the (4140) and the occipital region band portion (4120), and the upper left connecting band portion (4130) and the lower-left connecting band portion (4150). It may include a band portion (4160) and a jawbone right band portion (4160-1) which vertically connects the upper right band portion (4130-1) and the lower right band portion (4150-1). Also, the headband body portion (4100) may support multiple portions of the patient's head in various ways. In the embodiment, the headband body portion (4100) may be manufactured so that the strength of contact with the patient's head can be adjusted.

According to an additional aspect, the multiple fixed band portions (4200) may be fixed to band hangers' plurality (1110). In the embodiment, one end of multiple fixed band portions (4200) may be fixed to corresponding portions of the headband body portion (4100), respectively. The other end of multiple fixed band portions (4200) may be respectively inserted into grooves of multiple band hangers (1110). The other end of multiple fixed band portions (4200) may be fixed by multiple fixed band portions (4200) or a fixing surface (4210) formed on the headband body portion (4100). In an embodiment, a male part of velcro may be formed on the other end of multiple fixed band portions (4200), and a female part of velcro may be formed on the fixing surface (4210).

According to an additional aspect, it is possible to fix between the head of the patient and the single-element ultrasound transducer (1000) using the headband (4000). Using the headband (4000) to fix the single-element ultrasound transducer (1000) closely, the patient can slightly move the head during ultrasound treatment. Besides, since the single-element ultrasound transducer (1000) is mounted on the articulated operator (3000), the patient can comfortably receive treatment without feeling a burden on the single-element's weight and size ultrasound transducer (1000).

In the embodiment, multiple fixed band portions (4200) may be made of inelastic material. Accordingly, the single-element ultrasound transducer (1000) can be fixed without movement.

### [Description of Claim 7]

According to a further aspect, as shown in Drawing 4 and Drawing 2, the articulated operator (3000) includes a support (3100), a stand (3200) extending vertically from the support (3100), and one end of the stand (3200), and may include a free Refractive Arm (3300) that is bent around at least one joint shaft.

The support (3100) may support components included in the low intensity focused ultrasonic treatment device. The support (3100) may support a stand (3200), a free Refractive Arm (3300), a detaching area (3400), and a single-element ultrasound transducer (1000) coupled to the detaching area (3400). Multiple wheels (3500) is placed at the bottom of the support (3100) so that the low intensity focused ultrasonic treatment device can be moved.

The stand (3200) extends vertically from the support (3100) and may have a constant height. One end of the free Refractive Arm (3300) is coupled to an upper portion of the stand (3200) and maybe refracted around at least one articulated shaft. In an embodiment, the articulated shaft may be implemented as a rotation shaft. For example, two Arms are rotatably connected to a rotation shaft placed on the free Refractive Arm (3300). The two Arms may be bent at a predetermined angle concerning the rotation shaft. The two Arms can be rotated finely concerning the shaft of rotation by the user's manipulation.

### [Description of Claim 8]

As shown in Drawing 2, according to an additional aspect, the detaching area (3400) has one end rotatably coupled to the other end of the free Refractive Arm (3300). The other end is a single-element ultrasound transducer (1000) can be rotatably coupled. Accordingly, the medical practitioner can control the position and posture of the single-element transducer (1000) concerning the patient's head in more various ways by manipulating the detaching area (3400). In one embodiment, one end of the detaching area (3400) and the other end of the free Refractive Arm (3300) may be rotatably coupled concerning a first rotation shaft (not shown) placed of in the detaching area (3400). Besides, in an embodiment, the other end of the detaching area (3400) and the single-element ultrasound transducer (1000) may be rotatably coupled concerning the second shaft of rotation (not shown) placed in the detaching area (3400).

### [Description of Claim 9]

As shown in Drawing 2, according to an additional aspect, the detaching area (3400) includes a free Refractive Arm (3300), a ball stud 1(3411) fixed to the other end, and ball stud 1(3411). The socket space 1 (3412) that surrounds the ball and rotates in all directions concerning the ball surface, ball stud 2(3421) fixed to the single-element ultrasound transducer (1000), and ball stud 2(3421). It may include socket space 2 (3422) surrounding the included ball and rotating in all directions concerning the ball su rface.

The first plate included in the ball stud 1 (3411) of the detaching area (3400) may be fixed to the other end of the free Refractive Arm (3300). Ball 1 may be formed at the end of the first stud extending from the first plate. The socket space 1 (3412) included in the detaching area (3400) surrounds the ball 1 in the ball stud 1 (3411) and rotates in all directions concerning the ball 1 surface.

The second plate included in the ball stud 2 (3421) of the detaching area (3400) may be fixed to the upper surface of the transducer housing (1100). Ball 2 may be formed at the end of the stud 2 extending from the second plate. The socket space 2 (3422) included in the detaching area (3400) surrounds the second ball in the ball stud 2 (3421) and rotates in all directions concerning the ball 2 surface.

Accordingly, a medical practitioner can control the position and posture of the single-element transducer (1000) concerning the patient's head in various ways by manipulating the detaching area (3400). Besides, since the socket spaces, (3412) and (3422) and the ball studs (3411) and (3421) can be separated, a single-element ultrasound transducer (1000) having a specifically Focal length can be selectively mounted on a low intensity focused ultrasonic treatment device has.

### [Description of Claim 10]

According to an additional aspect, as shown in Drawing 4, the free Refractive Arm (3300) includes an Arm 1 (3310) rotatably coupled with one end thereof relative to the first vertical shaft of rotation to the upper portion of the stand (3200), Arm 2 (3320), whose one end is rotatable concerning the second vertical shaft of rotation to the other end of Arm 1 (3310) and rotatable concerning the first horizontal shaft of rotation. One end of Arm 2 (3320) may include an Arm 3 (3330) rotatably coupled to the other end of the second horizontal shaft of rotation.

In the embodiment, one end of the Arm 1 (3310) is rotatably coupled to a first vertical rotation shaft (not shown) placed inside the stand (3200) based on the first vertical rotation shaft according to the operation of the medical personnel. In the embodiment, Arm 1 (3310) may extend in an upward obtuse angle between 90 degrees and 180 degrees concerning the vertical stand (3200).

In an additional aspect, Arm 2 (3320) may be rotatably coupled to the other end of Arm 1 (3310) with one end of the second longitudinal shaft of rotation. One end of Arm 2 (3320) may be rotatably in an embodiment coupled to a second vertical rotation shaft placed inside the other end of Arm 1 (3310). Accordingly, Arm 2 (3320) may rotate left and right based on the second vertical rotation shaft according to the medical personnel's manipulation. Additionally, one end of Arm 2 (3320) may be rotatably coupled to the other end of Arm 1 (3310) concerning a first horizontal rotation shaft (not shown). In an embodiment, the first horizontal rotation shaft may be formed along a through-hole (not shown) formed in a horizontal direction on the second vertical rotation shaft placed inside the Arm 1 (3310) the other end. The second horizontal rotation shaft (not shown) may be inserted into the through-hole in the horizontal direction. Besides, both ends of the second horizontal rotation shaft may be fixed to the inner wall of Arm 2 (3320). Accordingly, Arm 2 (3320) may be rotated vertically based on the first horizontal rotation shaft according to the medical personnel's manipulation. In an embodiment, Arm 2 (3320) may extend in a downward acute angle between 1° and 90° concerning the second vertical rotation shaft.

Accordingly, Arm 1 (3310) and Arm 2 (3320) can rotate left and right around the second vertical shaft of rotation and rotate up and down around the first horizontal shaft of rotation. In a preferred embodiment, the medical personnel can adjust the height of the single-element ultrasound transducer (1000) by rotating the Arm 2 (3320) in the vertical direction. Besides, medical personnel rotate the first rotary Arm (3310) to the right concerning the first vertical shaft of rotation and rotate the second rotary Arm (3320) to the left concerning the second vertical shaft of rotation, for example, the position of the wave transducer (1000) can be adjusted in the rear direction (toward the stand (3200). Conversely, if the medical personnel rotate Arm 1 (3310) and Arm 2 (3320) so that the two Arms ((3310), (3320)) are in a straight line when viewed from above, the position of the single-element ultrasound transducer (1000) is moved in the omnidirectional direction. It can be adjusted to a position opposite to the stand (3200).

In an embodiment, Arm 3 (3330) may have one end rotatably coupled to the other end of Arm 2 (3320) concerning a second horizontal rotation shaft (not shown). One end of Arm 3 (3330) may be rotatably coupled to the second horizontal rotation shaft placed inside the other end of Arm 2 (3320). In an embodiment, when a medical practitioner moves Arm 2 (3320) upward or downward, Arm 3 (3330) rotates in the first direction (e.g., the stand (3200) direction) based on the second horizontal shaft of rotation. Alternatively, it can be rotated in a second direction (for example, in a direction opposite to the stand (3200)). Accordingly, Arm 3 (3330) can always maintain a vertical direction.

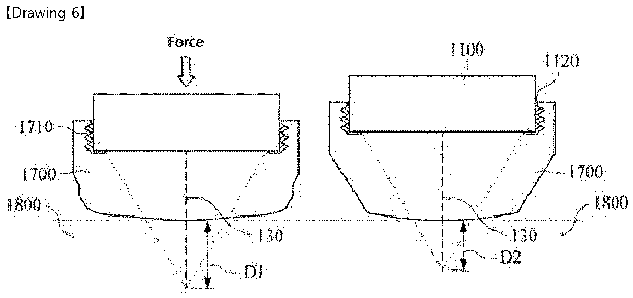

Drawing 6 is shows the state of use of a single-element ultrasound transducer according to a change in the appearance of a semi-solid ultrasound gel layer according to an embodiment. It will be described below in Drawing 6 and Drawing 2.

### [Description of Claim 11]

As shown in Drawing 6, the low intensity focused ultrasonic treatment device is placed in a detachable manner on the bottom of the single-element ultrasound transducer (1000) and the space between the patient's skin (1800)and the single-element ultrasound transducer (1000). It may further include an ultrasound gel layer (1700) for mediating ultrasound beam delivery within the space of the patient's skin (1800) and the single-element ultrasonic transducer (1000).

As is well known, when air exists between the patient's skin (1800) (e.g., the scalp) and the single-element ultrasound transducer (1000), the ultrasound beam is not properly transmitted to the patient's interior due to attenuation or the like. Accordingly, in an additional aspect, the ultrasound gel layer (1700) prevents a phenomenon in which space does not transmit the focused ultrasound beam between the single-element ultrasound transducer (1000) and the patient's skin. The ultrasound gel layer (1700) is formed of an ultrasound propagation mediating material (couplant) and serves to fill the space that may be generated.

As is known, the ultrasound gel layer (1700) can be implemented in a semi-solid type (refer to Korean Patent Publication No. 10-1585301) or a solid type (see Korean Patent Publication No. 20120118864). The ultrasound gel layer (1700), as disclosed in Korean Laid-Open Patent Publication No. 20120118864, is a solid form having elasticity and is inserted into the outer peripheral surface of the lower portion of the transducer housing (1100) of the single-element ultrasound transducer (1000). The inner space may be in the shape of an empty lid (or case, cover, etc.) so that it can be lost. Accordingly, the ultrasound gel layer (1700) may be placed in a detachable manner on the bottom of the single-element ultrasound transducer (1000).

Alternatively, as disclosed in Korean Patent Publication No. 10-1585301, the single-element ultrasound transducer (1000) and the transducer housing (1100) have a cylindrical shape when the shape of a single piezoelectric element (1200) provided therein (See Drawing 2). Further, a spiral protrusion (refer to 1120 of Drawings 6) may be formed on the lower outer surface of the transducer housing (1100). Besides, the ultrasound gel layer (1700) may have a shape of a lid (or case, cover, etc.) having an empty inside, and a spiral groove (see 1710 of Drawing 6) may be formed on the inner circumferential surface of the lid. Accordingly, the ultrasound gel layer (1700) may be placed of detachably using a helical coupling of the bottom of the single-element ultrasound transducer (1000). Accordingly, a specific ultrasound gel layer (1700) corresponding to any single-element ultrasound transducer (1000) corresponding to a specifically Focal length may be selected and placed detachably on the bottom of the single-element ultrasound transducer (1000).

### [Description of Claim 12]

As shown in Drawing 6, according to an additional aspect of the present invention, the ultrasound gel layer (1700) is applied to the external shape of the patient skin (1800) and the single-element ultrasound transducer (1000) by the user's manipulation. It is a semi-rigid type whose appearance is deformed in response to the magnitude and direction of the force (F) and the distance between the surface of the patient's skin (1800) and the area to be treated in the patient's skin (D2) and can be varied (D1>D2). In an embodiment, the ultrasound gel layer (1700) is a semi-rigid type. It has flexibility, elasticity, and compressibility, so that its appearance may be deformed according to the appearance of the patient's skin (1800). In the embodiment, the ultrasound gel layer (1700) may be implemented as a semi-rigid type hydrogel, as disclosed in Korean Patent Publication No. 10-1585301. As disclosed in the publication, the semi-solid hydrogel may be implemented with polyvinyl alcohol (PVA), which is hydrophilic without irritation to the human body.

Besides, in the embodiment, the semi-solid-type ultrasound gel layer (1700) may change its shape in response to the force's magnitude, and direction (F) applied to the single-element ultrasound transducer (1000) by the user's manipulation. According to this shape modification, the ultrasound gel layer (1700) may vary (D2>D1) the distance D between the surface of the patient's skin (1800) and the area to be treated in the patient's skin.

As shown in Drawing 6, the Focal length (130) does not change. However, before the shape of the semi-solid-type ultrasound gel layer (1700) is deformed by compression or the like, the distance between the surface of the patient's skin (1800) and the area to be treated in the patient's skin is D2. However, when the shape of the semi-solid-type ultrasound gel layer (1700) is deformed by compression or the like, the distance between the surface of the patient's skin (1800) and the area to be treated in the patient's skin changes to D1. The Focal length (130) does not change, but the single-element ultrasound transducer (1000) can treat a deeper treatment target area from the patient's skin (1800) with the help of the semi-solid type ultrasound gel layer (1700). There will be. In other words, the single-element ultrasound transducer (1000) can more precisely treat areas to be treated at various locations inside the patient's skin.

In the above, the present invention has been described concerning the accompanying drawings. Still, the present invention is not limited thereto, and it should be interpreted to encompass various modifications that can be apparently derived from those skilled in the art. The claims are intended to cover these variations.

**[Explanation of codes]**

| | | | |
|---|---|---|---|
| (1000) : | Single-element ultrasound transducer | (100) : | Focused region |
| (110) : | Maximum acoustic intensity point | (120) : | Focused region diameter |
| (130) : | Focal length | (200) : | Focused ultrasound beam |
| (1100) : | Transducer housing | (1110) : | Multiple band hanger |
| (1120) : | Spiral protrusion | (1200) : | Single piezoelectric element |
| (1300) : | Spherical lens | (1400) : | Electrode |
| (1500) : | Connector | (1600) : | Connector cover |
| (1700) : | Ultrasound gel layer | (1710) : | Spiral groove |
| (1800) : | Patient's skin | (2000) : | Transducer marker |
| (3000) : | Articulated operator | (3100) | : Support |
| (3200) : | Stand | (3300) : | free Refractive Arm |
| (3310) : | Arm 1 | (3320) : | Arm 2 |
| (3330) : | Arm 3 | (3400) : | Detaching area |
| (3410) : | Ball joint 1 | (3411) : | Ball stud 1 |
| (3412) : | Socket space 1 | (3420) : | Ball joint 2 |
| (3421) : | Ball stud 2 | (3422) : | Socket space 1 |
| (3500) : | Multiple wheels | (4000) : | Headband |
| (4100) : | Headband body portion | (4110) : | Forehead band |
| (4120) : | occipital region band portion | (4130) : | Left top connection band |
| (4140) : | Lower jawbone band | (4150) : | Left bottom connection band |
| (4150-1) : | Right bottom connection band | (4160) : | Left jawbone band |
| (4160-1 : | Jawbone right band portion | (4200) : | Multiple fixed band |

## Claims

1. The focal area diameter at the point of maximum acoustic intensity in the focal region is 6 mm to 9 mm, and the focal length from the surface to the point of maximum acoustic intensity is a single element ultrasonic transducer that outputs a low intensity focused ultrasonic beam of 30mm to 80mm and a transducer marker that provides position and posture information of the single-element ultrasound transducer fixed to the single-element ultrasound transducer.

2. In Claim 1;
A low-intensity focused ultrasound treatment apparatus in which the sound intensity in the focused area of the ultrasound beam output from the single-element ultrasound transducer is 50 watts per square centimeter (50 W/cm²) or less.

3. In Claim 2;
The single-element ultrasonic transducer has an electrode that applies voltage, a single piezoelectric element that outputs a low-intensity ultrasonic beam, a spherical lens that focuses a low-intensity ultrasonic beam, and a connector and electrode that electrically connects an external control cable and electrode A low-intensity focused ultrasound treatment apparatus comprising a transducer housing for arranging a piezoelectric element, a spherical lens, and a connector.

4. In any one of Claims 1 to 3;
Low-intensity focused ultrasound treatment apparatus further comprising a multi-joint manipulation unit coupled to the single-element ultrasonic transducer so that the ultrasonic treatment device can adjust the position and posture of the single-element ultrasonic transducer.

5. In Claim 4;
The ultrasound treatment device includes multiple single-element ultrasound transducers with different Focal lengths, and a low intensity focused ultrasonic treatment that includes a detaching area that couples one of multiple single-element ultrasound transducers in a detachable manner to the articulated operator.

6. In Claim 4;
An ultrasound treatment device comprising multiple band hangers formed along the edge of a single-element ultrasound transducer and multiple bands fixed while wrapping the patient's head between multiple band hangers.

7. In Claim 4;
The articulated operator is a low intensity focused ultrasonic treatment device comprising a stand extending vertically from the support and support, and a free Refractive Arm has one end coupled to the upper part of the stand and bendable around at least one joint shaft.

8. In Claim 7;
The detaching area is a low intensity focused ultrasonic treatment device whose one end is rotatably coupled to the other end of the free Refractive Arm. The other end is rotatably coupled to a single-element ultrasound transducer.

9. In Claim 8;
The detaching area includes a ball stud 1 fixed to the free Refractive Arm, the other end, a socket space 1 surrounding a ball included in the ball stud 1 and rotating in all directions concerning the ball surface, and a single-element, a low intensity focused ultrasonic treatment device comprising ball stud 2 fixed to an ultrasound transducer, and the socket space 2 surrounding a ball is included in the ball stud 2 and rotating in all directions concerning the ball surface.

10. In Claim 7;
Arm 1 in which one end of the free Refractive Arm is rotatably coupled to the top of the stand concerning the first vertical shaft of rotation, and the other end of the free Refractive Arm is rotatable concerning the second longitudinal shaft of rotation in the other end of the Arm 1 and the first horizontal shaft of rotation Arm 2 and rotatably coupled based on; and a low intensity focused ultrasonic treatment device including Arm 3 whose one end is rotatably coupled to the other end of Arm 2 based on a second horizontal rotation shaft.

11. In Claim 5;
A low intensity focused ultrasonic treatment device comprising an ultrasound gel layer that is detachably placed on the bottom of the single-element ultrasound transducer and mediates ultrasound beam delivery within the space between the patient's skin and the single-element ultrasound transducer.

12. In Claim 11;
The ultrasound gel layer is a semi-rigid type whose appearance is deformed in response to the force's size and direction applied to the single-element ultrasound transducer by the appearance of the patient's skin and the user's manipulation, and a low intensity focused ultrasonic treatment device changes the distance between the patient's skin surface and the area to be treated in the patient's skin as its shape changes.
